(19) Europäisches Patentamt
European Patent Office
Office européen des brevets

(11) **EP 3 869 519 A2**

(12) **EUROPEAN PATENT APPLICATION**
published in accordance with Art. 153(4) EPC

(43) Date of publication:
**25.08.2021 Bulletin 2021/34**

(21) Application number: **19873870.0**

(22) Date of filing: **23.09.2019**

(51) Int Cl.:
***G16H 50/20*** (2018.01)        ***A61B 5/00*** (2006.01)

(86) International application number:
**PCT/KR2019/012350**

(87) International publication number:
**WO 2020/080689 (23.04.2020 Gazette 2020/17)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **15.10.2018 KR 20180122851**

(71) Applicant: **Lookinbody Inc.**
**Seoul 06313 (KR)**

(72) Inventors:
• **KIM, Hyun Jae**
**Seoul 06313 (KR)**
• **KIM, Hyun Seok**
**Seoul 06313 (KR)**
• **KANG, Dong Woo**
**Seoul 06313 (KR)**

(74) Representative: **Morrall, Jonathan Ian McLachlan
et al**
**Kilburn & Strode LLP**
**Lacon London**
**84 Theobalds Road**
**London WC1X 8NL (GB)**

(54) **APPARATUS AND METHOD FOR GENERATING INDEX VALUE FOR COMPARING BODY COMPOSITION CHANGE PERFORMANCE**

(57)    Provided is a computing device for generating an index value for comparing the body composition change performance between users having different body compositions. The computing device can comprise: a communication unit for acquiring body composition data of a user from a connected body composition measurement device or updating a body composition change amount of the user for a first period of time; a processor for calculating a second period of time required for a pre-selected user group in order to achieve the body composition change amount; and an output unit for generating an index value for comparing the body composition change performance between users having different body compositions by using the first period of time and the second period of time.

FIG. 6

**Description**

Technical Field

The following description relates to an apparatus and method for generating index values for comparing body composition change performances between users having different body compositions.

Background Art

With an increasing population who pursue the values of life such as well-being or lifestyles of health and sustainability (LOHAS), the healthcare market is also steadily growing. Today, people measure their body composition changes using body composition measurement devices placed at fitness centers and the like, and are provided with analysis on health conditions through the results of measurements.

However, the related arts could not provide an objective comparison regarding who did weight control well and who did not, when a plurality of users performed weight control for the same period. That is because the users have different initial body composition states and physical characteristics such as gender, age, height, weight, body fat mass, and skeletal muscle mass. For example, even when two different users had the same reduction in body fat mass, the users must have required different times or efforts to achieve the body composition changes according to the personal characteristics such as the initial body fat mass. Thus, there is a need for calculating their performances differently.

An invention relating to a method and apparatus for measuring physical fitness for determining a basic physical condition of a user is disclosed in Korean Patent No. 1470593. Specifically, the patent includes a feature for calculating an overall fitness index of a user by applying predetermined weights to body compositions and physical strength elements such as cardiorespiratory endurance, muscular strength, muscular endurance, balance, and flexibility. However, it fails to disclose any technical ideas about how to compare the body composition changes of different users.

Disclosure of Invention

Technical Goals

An aspect provides an apparatus and method for generating an index value for objectively comparing body composition change performances between users having different body compositions and physical characteristics.

Technical solutions

According to an aspect, there is provided a computing device for generating an index value for comparing body composition change performances between users having different body compositions. The computing device may include a communication unit configured to obtain body composition data of a user from a body composition measurement apparatus that is in connection thereto or to update a body composition change of the user achieved for a first period, a processor configured to calculate a second period required for a pre-selected user group to achieve the body composition change, and an output unit configured to generate an index value for comparing body composition change performances between users having different body compositions based on the first period and the second period.

According to an example embodiment, the processor may be configured to select a first user group having a body fat decrease or a muscle mass increase in a pre-selected storage device, and to calculate a second user group in the first user group as the pre-selected user group, the second user group whose age, gender, and height have a predetermined or higher correlation with those of the user.

According to another example embodiment, the processor may be configured to calculate, as the second period, a period required to reach a body fat mass and a muscle mass after the first period from a body fat mass and a muscle mass of the user before the first period, based on body composition change data of the second user group.

According to still another example embodiment, when the output unit is implemented as a display, the display may be configured to display the generated index value on a screen.

According to yet another example embodiment, when the output unit is implemented as a printer, the printer may be configured to provide a result printout including the generated index value.

According to another aspect, there is provided a method of displaying an index value for comparing body composition change performances between users having different body compositions. The method may include transmitting an instruction signal to an output device such that the output device displays an index value for comparing body composition change performances between users having different body compositions. The index value may be calculated based on a first period corresponding to a body composition change of a user and a second period required for a pre-selected user group to achieve the body composition change.

According to an example embodiment, the instruction signal may include any one of a first instruction signal to display a pre-calculated index value, a second instruction signal including at least one of the first period and the second period and to calculate the index value, and a third instruction signal including the first period and to calculate the second period corresponding to the first period.

According to another example embodiment, the second period may be calculated as a period required for the pre-selected user group to reach a body fat mass and a muscle mass after the first period from a body fat mass and a muscle mass of the user before the first period, the pre-selected user group may be pre-selected as a second user group whose age, gender, and height have a predetermined or higher correlation with those of the user, in a first user group having a body fat decrease or a muscle mass increase, and a value obtained by dividing the second period by the first period may be calculated as the index value.

According to still another example embodiment, the method may include obtaining body composition data of the user according to a pre-defined data transaction scheme, updating a body composition change of the user achieved for the first period based on the obtained body composition data, and calculating, as the index value, a value obtained by dividing the second period by the first period.

According to yet another example embodiment, the method may further include calculating, as the second period, a period required for the pre-selected user group to reach a body fat mass and a muscle mass after the first period from a body fat mass and a muscle mass of the user before the first period.

According to still another example embodiment, the obtaining of the body composition data of the user may include any one of receiving the body composition data of the user from a body composition measurement apparatus that is in connection, receiving the body composition data of the user being inputted through a user interface, and extracting the body composition data of the user from a designated code image.

According to still another aspect, there is provided a body composition measurement apparatus for generating an index value for comparing body composition change performances of users having different body compositions. The body composition measurement apparatus may include a user identifier configured to identify a user whose body composition is to be measured through the body composition measurement apparatus based on user information being inputted through a user interface, an electrode unit configured to measure current body composition data of the identified user, a memory configured to store body composition data of a plurality of users measured for a predetermined period, a processor configured to update a body composition change of the identified user achieved for a first period based on previous body composition data and the current body composition data of the identified user received from the memory and to calculate a second period required for a pre-selected user group to achieve the body composition change, and an output unit configured to generate an index value for comparing body composition change performances between users having different body compositions based on the first period and the second period.

According to yet another aspect, there is provided a computing device for generating an index value for comparing body composition change performances between users having different body compositions. The computing device may include a communication unit configured to obtain body composition data of a user from a body composition measurement apparatus that is in connection thereto or to update a first body composition change of the user achieved for a first period, a processor configured to calculate a second body composition change predicted to be achievable by a pre-selected user group for the first period according to a big data analysis result, and an output unit configured to generate an index value for comparing body composition change performances between users having different body compositions based on the first body composition change and the second body composition change.

According to an example embodiment, the output unit may be configured to generate, as the index value, a value obtained by dividing the first body composition change by the second body composition change.

According to still another aspect, there is provided a method of displaying an index value for comparing body composition change performances between users having different body compositions. The method may include transmitting an instruction signal to an output device such that the output device displays an index value for comparing body composition change performances between users having different body compositions. The index value may be calculated based on a first body composition change of a user achieved for a first period and a second body composition change predicted to be achievable by a pre-selected user group for the first period.

According to an example embodiment, the instruction signal may include any one of a first instruction signal to display a pre-calculated index value, a second instruction signal including at least one of the first body composition change and the second body composition change and to calculate the index value, and a third instruction signal including the first body composition change and to calculate the second body composition change based on the first body composition change.

According to another example embodiment, the index value may be calculated as a value obtained by dividing the first body composition change by the second body composition change.

According to still another example embodiment, the method may include obtaining body composition data of the user according to a pre-defined data transaction scheme, updating the first body composition change of the user achieved for the first period based on the received body composition data and, calculating, as the index value, a value obtained by dividing the second body composition change by the first body composition change.

According to yet another aspect, there is provided a body composition measurement apparatus for generating an index value for comparing body composition change performances of users having different body compositions. The body composition measurement apparatus may include a user identifier configured to identify a user whose body composition is to be measured through the body composition measurement apparatus based on user information being inputted through a user interface, an electrode unit configured to measure current body composition data of the identified user, a memory configured to store body composition measurement values of a plurality of users measured for a predetermined period, a processor configured to update a first body composition change of the identified user achieved for a first period based on previous body composition data and the current body composition data of the identified user received from the memory and to calculate a second body composition change predicted to be achievable by a preselected user group for the first period according to a big data analysis result, and an output unit configured to generate an index value for comparing body composition change performances between users having different body compositions based on the first body composition change and the second body composition change.

Brief Description of Drawings

FIG. 1 is a block diagram of an apparatus for generating an index value for comparing a body composition change performance according to an example embodiment.
FIGS. 2A and 2B are exemplary diagrams illustrating a process of calculating an index value by an index value generating apparatus according to another example embodiment.
FIG. 3 is an exemplary diagram illustrating a process of calculating an index value by an index value generating apparatus according to still another example embodiment.
FIG. 4 is an exemplary diagram illustrating a process of calculating an index value by an index value generating apparatus if a plurality of body compositions changes according to still another example embodiment.
FIG. 5 is an exemplary diagram illustrating diverse examples of obtaining body composition data by an index value generating apparatus.
FIG. 6 illustrates a user interface displaying index values according to an example embodiment.
FIG. 7 illustrates a body composition measurement apparatus for generating an index value according to an example embodiment.

Best Mode for Carrying Out the Invention

The following detailed structural or functional description is provided as an example only and various alterations and modifications may be made to the example embodiments. Accordingly, the example embodiments are not construed as being limited to the disclosure and should be understood to include all changes, equivalents, and replacements within the technical scope of the disclosure.

Terms, such as first, second, and the like, may be used herein to describe components. Each of these terminologies is not used to define an essence, order or sequence of a corresponding component but used merely to distinguish the corresponding component from other component(s). For example, a first component may be referred to as a second component, and similarly the second component may also be referred to as the first component.

It should be noted that if it is described that one component is "connected", "coupled", or "joined" to another component, a third component may be "connected", "coupled", and "joined" between the first and second components, although the first component may be directly connected, coupled, or joined to the second component.

The singular forms "a", "an", and "the" are intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms, including technical and scientific terms, used herein have the same meaning as commonly understood by one of ordinary skill in the art to which this disclosure pertains. Terms, such as those defined in commonly used dictionaries, are to be interpreted as having a meaning that is consistent with their meaning in the context of the relevant art, and are not to be interpreted in an idealized or overly formal sense unless expressly so defined herein.

Hereinafter, example embodiments will be described in detail with reference to the accompanying drawings. When describing the example embodiments with reference to the accompanying drawings, like reference numerals refer to like components and a repeated description related thereto will be omitted.

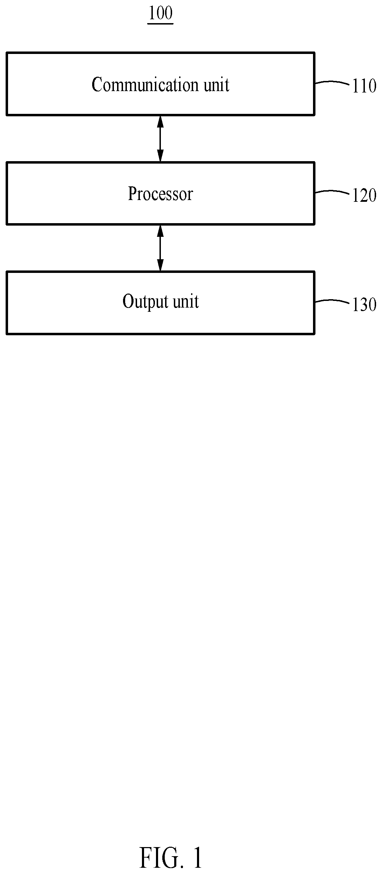

FIG. 1 is a block diagram of an apparatus for generating an index value for comparing a body composition change performance according to an example embodiment. An index value generating apparatus 100 may generate an index value for comparing body composition change performances between users having different body compositions. More specifically, the index value generating apparatus 100 may generate a first index value for objectively comparing body composition change paces between different users. In addition, the index value generating apparatus 100 may generate a second index value for objectively comparing body composition change rates between different users based on body composition target values.

Referring to FIG. 1, the index value generating apparatus 100 may include a communication unit 110, a processor 120, and an output unit 130. For example, the index value generating apparatus 100 may be implemented in the form of a computing device or user terminal that generates an index value. The communication unit 110 may obtain body composition data from a body composition measurement apparatus that is in connection thereto through a communication interface. Specifically, the communication unit 110 may be implemented in the form of a communication module including a communication interface. For example, the communication interface may include wireless Internet interfaces such as a wireless LAN (WLAN), wireless-fidelity (Wi-Fi) Direct, Digital Living Network Alliance (DLNA), wireless broadband (WiBro), Worldwide Interoperability for Microwave Access (WiMAX), and High Speed Downlink Packet Access (HSDPA), and short-range communication interfaces such as Bluetooth™, radio-frequency identification (RFID), Infrared Data Association (IrDA), ultra-wideband (UWB), ZigBee, and Near-Field Communication (NFC). In addition, the communication interface may include all interfaces (for example, wired interfaces) capable of external communication.

The communication unit 110 may update a body composition change of a user achieved for a first period based on the obtained body composition data. As an example, the communication unit 110 may obtain the body composition data from the body composition measurement apparatus that is in connection thereto, and calculate and update the body composition change achieved for the first period by comparing the obtained body composition data to previous body composition data. As another example, the communication unit 110 may receive the body composition change achieved for the first period from the body composition measurement apparatus having a storage function. In the following description, the first period may be a temporal interval between a first point in time at which body composition data of a user is measured and a second point in time at which body composition data of the user is measured again after a predetermined time elapses.

The processor 120 may calculate a second period required for a pre-selected user group to achieve the body composition change of the user. Specifically, the processor 120 may select a first user group having a body fat mass decrease or a skeletal muscle mass increase in a pre-selected storage device. The storage device may be implemented in the form of a memory device such as a hard disk or a universal serial bus (USB) as well as a network connection-based storage device such as a cloud server.

Further, the processor 120 may extract, from the first user group, a second-first user group having a predetermined or higher correlation based on the age of the user, a second-second user group having a predetermined or higher correlation based on the gender of the user, and a second-third user group having a predetermined or higher correlation based on the height of the user. In addition, the processor 120 may calculate users belonging in common to the second-first user group, the second-second user group, and the second-third user group as the pre-selected user group (= second user group).

The processor 120 may calculate, as the second period, a period required for the second user group to achieve a body fat mass change and/or a skeletal muscle mass change achieved by the user for the first period according to a big data analysis based on body composition change data of the calculated second user group.

The output unit 130 may generate the index value for comparing body composition change performances of users having different body compositions based on the first period and second period calculated by the processor 120. The output unit 130 may be implemented in the form of an output device for displaying a result of processing transacted data to a user.

As an example, if the output unit 130 is implemented as a display, the display may display the generated index value on a screen, thereby showing the user a body composition change performance. As another example, if the output unit 130 is implemented as a printer, the printer may provide a result printout including the generated index value. The above description of the display and the printer is provided for better understanding and merely exemplary and thus, should not be construed as limiting or restricting other example embodiments. For example, an example embodiment of implementing the output unit 130 as a speaker for outputting the body composition change performance of the user through an audio signal may also be within the spirit of the invention.

As another example, the index value generating apparatus 100 may calculate the first index value and the second index value based on the body composition change. The communication unit 110 may update a first body composition change of the user achieved for the first period based on the body composition data received from the body composition measurement apparatus that is in connection thereto. The processor 120 may calculate a second body composition change predicted to be achievable by the pre-selected user group for the first period according to a big data analysis result. The output unit 130 may generate the index value for comparing body composition change performances of users having different body compositions based on the first body composition change and the second body composition change. Specifically, the output unit 130 may calculate, as the first index value, a value obtained by dividing the first body composition change by the second body composition change.

The processor 120 may select the first user group having a body fat mass decrease or a skeletal muscle mass increase in the pre-selected storage device. In addition, the processor 120 may calculate the second user group in the first user group as the pre-selected user group, the second user group whose age, gender, and height have the predetermined or higher correlation with the user.

As another example, the processor 120 may calculate a third body composition change predicted to be achievable by the pre-selected user group for a third period according to the big data analysis result, wherein the third period is the whole weight control period set for the user. For example, the third body composition change may include body composition changes for a plurality of body composition parameters, such as a body fat mass, a skeletal muscle mass, and the like. The output unit 130 may generate the index value for comparing body composition change performances of users having different body compositions based on the first body composition change and the third body composition change. Specifically, the output unit 130 may calculate, as the second index value, a value obtained by dividing the first body composition change by the third body composition change.

In the examples described with reference to FIG. 1, the process of obtaining body composition data, directly calculating the first period and the second period, and generating the index value based on the calculated first and second periods by the index value generating apparatus 100 is described. However, the configuration of calculating the index value in the process of implementing the present example embodiment may be implemented to be processed in a distributed manner through a plurality of entities and finally transmit an instruction signal for outputting the index value. Hereinafter, the example embodiment will be described in more detail.

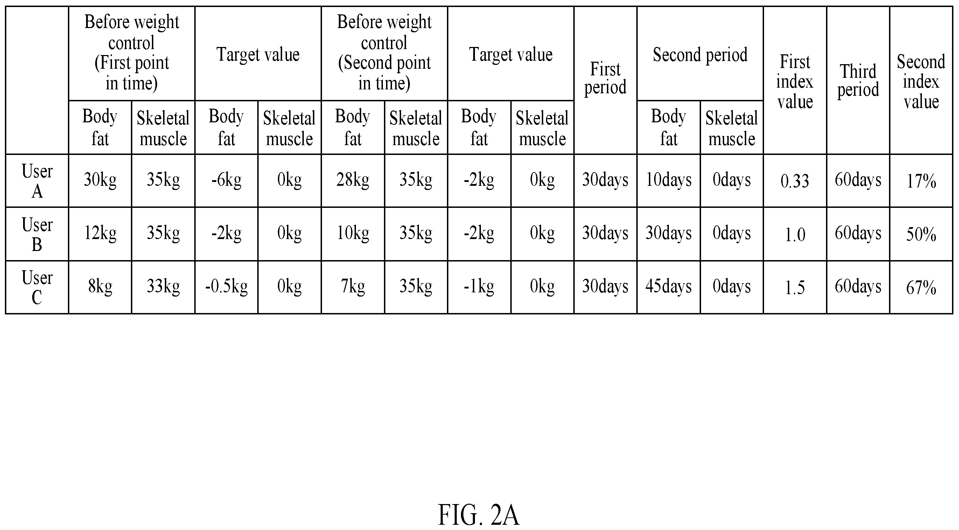

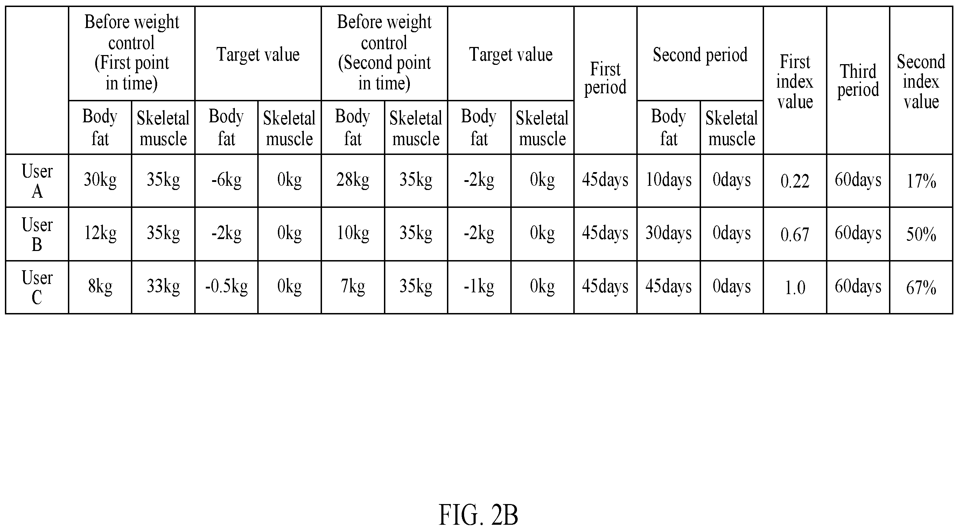

FIGS. 2A and 2B are exemplary diagrams illustrating a process of calculating an index value by an index value generating apparatus according to another example embodiment. Referring to FIG. 2A, a process of calculating first index values and second index values based on body composition data of users A, B, and C having different body compositions is illustrated.

The process of calculating the first index values and the second index values may be performed directly by a body composition measurement apparatus, or may be performed through a server that operates an application or web service for outputting a body composition change performance or through a user terminal or computer that executes the application or web service. In addition, processing the process of a series of operations in a distributed manner through a plurality of entities and generating an instruction signal for outputting the first index values and the second index values, which are the final results, by transmitting and receiving only the results of transacting the processed data should also be construed as being within the scope of the present example embodiment.

In the example of FIG. 2A, a first period may be a body composition change period of a user during which more than two body composition measurements are performed. Specifically, FIG. 2A describes a case in which all the users A, B, and C spend the same period of 30 days from a first point in time before the weight control to a second point in time at which the weight control is continued. For the first period which is the period during which the weight control was performed actually, the user A reduced 2 kg of body fat, the user B also reduced 2 kg of body fat, and the user C reduced 1 kg of body fat. In this case, an index value generating apparatus may calculate second periods corresponding to the respective users. Specifically, a second period may be a weight control required period that is calculated through big data analysis from a user group matched with a predetermined user. That is, the index value generating apparatus may extract a user group sharing a predetermined or higher correlation with the predetermined user with respect to a personal characteristic through the big data analysis, and calculate, as the weight control required period, a high-confidence second period by performing a probability and statistical analysis on the extracted result (for example, a pre-selected user group).

Further, when the user shows a dissatisfactory body composition change based on initial body composition data, the weight control required period may be calculated at a negative value as a period required to restore the increased body fat mass to its original state.

According to this principle, although the user A and the user B have the same body composition change, the second period for the user A may be predicted to be 10 days, and the second period for the user B may be predicted to be 30 days. Likewise, the index value generating apparatus may predict the second period for the user C to be 45 days based on the big data analysis. The index value generating apparatus may calculate a first index value associated with a body composition change pace of a user according to Equation 1 below.

[Equation 1]

$$\text{First index value} = \frac{\text{Second period which is weight control required period}}{\text{First period which is actual weight control period}}$$

In Equation 1, to help understanding, the first period has been described as the actual weight control period, and the second period has been described as the weight control required period. However, it is obvious to those skilled in the art to use an actual body composition change period based on a skeletal muscle mass increase and a body water mass decrease as the first period and a body composition change required period as the second period, which is within the idea of the present example embodiment.

The index value generating apparatus may predict 10 days as a period required for a user group associated with the user A to reduce 2 kg of body fat, based on the initial body composition and the personal characteristic of the user A. In this case, since the user A has achieved a body composition change corresponding to the second period of 10 days for the first period of 30 days, the index value generating apparatus may calculate the first index value for the body composition change pace to be 0.33.

As another example, the index value generating apparatus may generate the second index values indicating body composition change rates with respect to reference target values. Referring to FIG. 2A, third periods corresponding to the respective users are shown. A third period may be a period predetermined according to user identification information, a period inputted by an expert such as a trainer, or a predicted period required for a pre-selected user group to achieve a target value. In this case, the index value generating apparatus may calculate a second index value associated with a body composition change rate of a user according to Equation 2 below.

[Equation 2]

$$\text{Second index value} = \frac{\text{Second period which is weight control required period}}{\text{Third period which is whole weight control period}}$$

For example, the user A achieved a body composition change corresponding to the second period of 10 days compared to the third period (60 days), which is the preset whole weight control period. Thus, the second index value indicating a body composition change rate may be calculated at 10/60, that is, about 17%.

FIG. 2B shows a case in which another 15 days elapses while all the users do not show special body composition changes, to clearly describe the meaning of the first index value and the second index value proposed herein. The user A failed to achieve a further body composition change during the first period of 45 days and achieved a body composition change corresponding to the second period of 10 days. Thus, the index value generating apparatus may calculate the first index value for the body composition change pace to be 10/45, that is, about 0.22. However, since the user A achieved only a body composition change corresponding to the second period of 10 days against the third period (60 days), which is the preset whole weight control period, the second index value indicating a body composition change rate may be calculated at 10/60, that is, about 17%, which may be fixed as in the example of FIG. 2A. It is obvious to those skilled in the art that the first index values of the other users B and C may be newly calculated according to the same principle.

According to the present example embodiment, the index value generating apparatus may more accurately compare weight control performances by outputting first index values for comparing, between different users, the weight control paces observed thus far to achieve weight control goals, and second index values indicating the degrees of weight control completed compared to the final weight control targets.

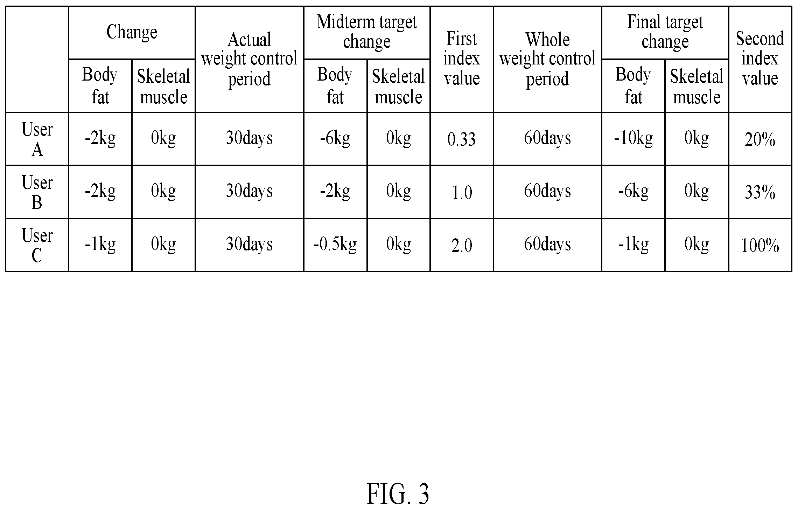

FIG. 3 is an exemplary diagram illustrating a process of calculating an index value by an index value generating apparatus according to still another example embodiment. Referring to FIG. 3, a process of calculating first index values and second index values based on body composition data of users A, B, and C having different body compositions is illustrated. Compared to the example embodiments of FIGS. 2A and 2B, a body composition measurement apparatus of the present example embodiment may generate index values for comparing weight control paces between users having different body compositions by predicting and tracking body composition changes rather than time periods.

The example embodiment of FIG. 2A also describes a case in which all the users A, B, and C spend the same period of 30 days from a first point in time before the weight control to a second point in time at which the weight control is continued. During the actual weight control period of 30 days, the user A reduced 2 kg of body fat, the user B also reduced 2 kg of body fat, and the user C reduced 1 kg of body fat. Further, the present example embodiment describes a case in which the whole weight control period is 60 days, and the users are in the weight control.

The index value generating apparatus may extract a user group sharing a predetermined or higher correlation with a predetermined user with respect to a personal characteristic through a big data analysis, and calculate a final target change corresponding to the whole weight control period and a midterm target change corresponding to the actual weight control period according to statistical value for the extracted result (for example, a pre-selected user group). Specifically, the index value generating apparatus may calculate that the user A needs to reduce 6 kg on and from the 30th day to reduce 10 kg of body fat mass during the whole weight control period of 60 days. The index value generating apparatus may calculate a midterm target change that more fits each user by setting the midterm target change based on the big data analysis, rather than an arithmetic calculation, based on the final target change.

The index value generating apparatus may calculate a first index value associated with a body composition change pace of a user according to Equation 3 below.

[Equation 3]

$$\text{First index value} = \frac{\text{Actual body composition change}}{\text{Midterm target change}}$$

As this principle, since the user A should have reduced 6 kg of body fat mass on and from the 30th day according to midterm target change but actually reduced only 2 kg, the index value generating apparatus may calculate the first index value for the body composition change pace to be 0.33.

Further, the index value generating apparatus may calculate a second index value associated with a body composition change rate of a user according to Equation 4 below.

[Equation 4]

$$\text{Second index value} = \frac{\text{Actual body composition change}}{\text{Final target change}}$$

In this case, since the user A actually reduced only 2 kg of body fat mass although the final target change is to reduce 10 kg of body fat during the whole weight control period of 60 days, the second index value may be calculated to be 20%. As described above, the index value generating apparatus may output first index values for more accurately comparing the weight control paces observed thus far and output second index values indicating the progress of the weight control to the final weight control goals according to midterm target changes and final target changes that are predicted by using a user group selected according to a big data analysis based on the current body composition states of the users.

In the example embodiments described above, for better understanding, the process of generating an index value when one of a plurality of body composition parameters of the user (for example, body fat mass) changes has been described. Hereinafter, a process of calculating a first index value and a second index value when a plurality of body composition parameters of a user change simultaneously will be described.

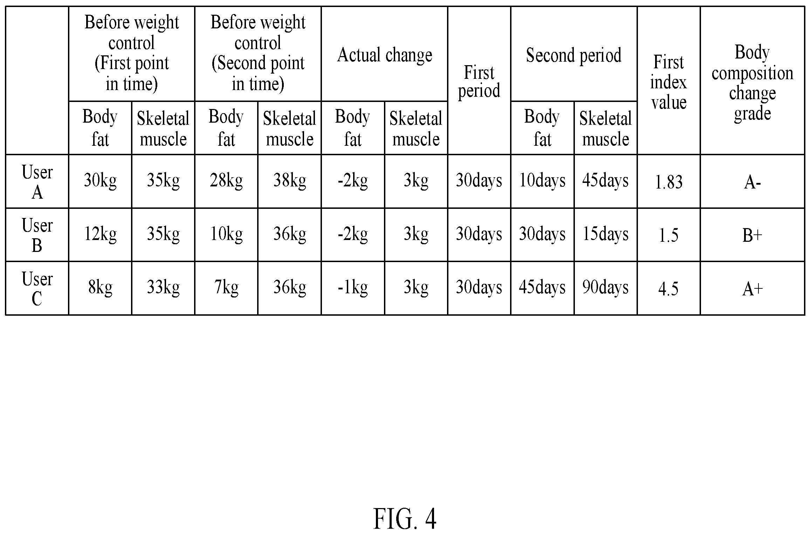

FIG. 4 is an exemplary diagram illustrating a process of calculating an index value by an index value generating apparatus if a plurality of body compositions changes according to still another example embodiment. In the example embodiment of FIG. 4, all the users A, B, and C changed their body compositions for 30 days (the first period) from a first point in time before they started the weight control to a second point in time at which they are in the weight control. Specifically, during the actual weight control period of 30 days, the user A reduced 2 kg of body fat and increased 3 kg of skeletal muscle mass, and the user B also reduced 2 kg of body fat and increased 3 kg of skeletal muscle mass. Further, the user C reduced 1 kg of body fat and increased 3 kg of skeletal muscle mass. The index value generating apparatus may extract a user group corresponding to the user A based on a personal characteristic (gender or height) and initial body composition data of the user A. Further, the index value generating apparatus calculates, using a big data analysis result including body composition changes of the extracted user group, that a pre-selected user group requires a weight control period of 10 days to reduce 2 kg of body fat and a weight control period of 45 days to increase 3 kg of skeletal muscle. As a result, the index value generating apparatus may calculate a total weight control required period of each user based on the required periods (second periods) corresponding to the plurality of body composition parameters. Since the user A achieved body composition changes of the plurality of body composition parameters corresponding to 55 days, which are the second period (the weight control required period), during the first period (the actual weight control period) of 30 days, the first index value for the body composition change pace may be calculated to be 55/30, that is, about 1.83, according to Equation 1. Further, since the user B achieved body composition changes corresponding to 45 days, which are the second period, during the first period of 30 days, the first index value for the body composition change pace may be calculated to be 45/30, that is, about 1.5.

The index value generating apparatus may predict periods required for a pre-selected user group matched with the user to achieve predetermined body composition changes based on the big data analysis. The required periods may be predicted for the respective body composition parameters. For example, fewer resources are required to reduce body fat if body fat mass is large, whereas more resources are required to reduce the same body fat if body fat mass is small. The index value generating apparatus may calculate weight control required periods from user groups having a predetermined or higher correlation with the personal characteristics and body composition specifications of the users, and generate index values for the body composition change paces and body composition change rates, thereby comparing body composition change performances between users having different body compositions.

Further, the index value generating apparatus may also generate the index values based on effort scores or effort indices corresponding to the actual body composition changes of the users. For example, an effort score or effort index may be a score or index required for a user group to achieve a predetermined body composition change according to a predesignated condition.

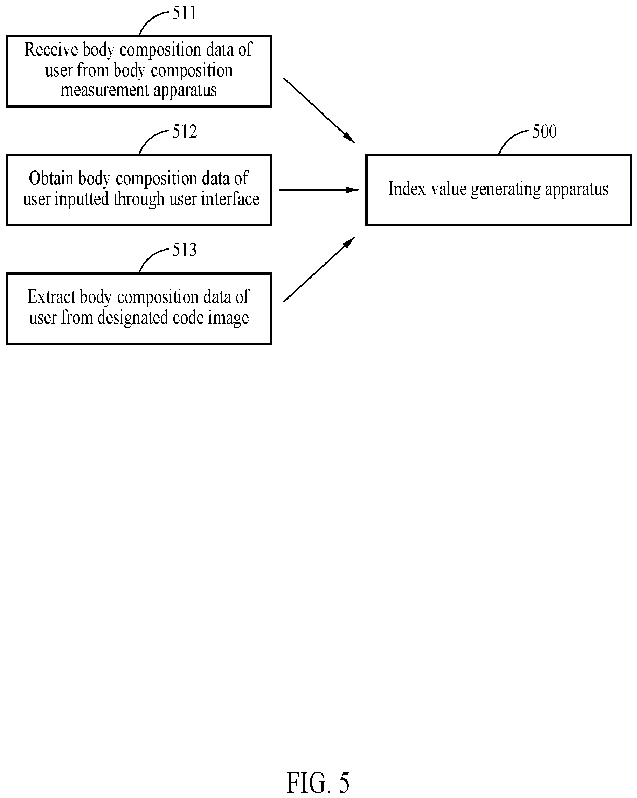

FIG. 5 is an exemplary diagram illustrating diverse examples of obtaining body composition data by an index value generating apparatus. Referring to FIG. 5, an index value generating apparatus 500 may obtain a body composition data of a user based on various example embodiments 511, 512, and 513. The index value generating apparatus 500 may obtain the body composition data of the user according to a predefined data transaction scheme. The predefined data transaction scheme will be described in more detail below.

According to an example embodiment 511, the index value generating apparatus 500 may receive the body composition data of the user from a body composition measurement apparatus that is in connection thereto. The index value generating apparatus 500 and the body composition measurement apparatus may transmit and receive data to and from each other through a communication interface. The description provided above may apply to the communication interface, and thus a detailed description will be omitted.

According to another example embodiment 512, the index value generating apparatus 500 may obtain the body composition data of the user inputted through a user interface. For example, the user interface may be implemented as a graphical object on a user terminal. The user may manually input the body composition data through an input device such as a mouse or a keyboard. Further, the user may input the body composition data by touching a display screen of the user terminal.

According to still another example embodiment 513, the index value generating apparatus 500 may extract the user body composition data from a designated code image. For example, the designated code image may be an image that is implemented as an informative code and printed out on a printout including body composition data of a predetermined user, such as a barcode image or quick response (QR) code image. The index value generating apparatus 500 may obtain the body composition data of the user by scanning the code image.

The above example embodiments of obtaining the body composition data are provided for better understanding and merely exemplary and thus, should not be construed as limiting or restricting other example embodiments. The method of obtaining the body composition data by the index value generating apparatus 500 may include techniques that can be derived at the time of filing a patent application and even a range of future equivalent substitutes. For example, the method of obtaining the body composition data, used in the process of implementing the index value generating apparatus 500, may include a scheme of estimating the body composition data based on body images or a scheme of reading out the body composition data based on an optical character reader (OCR).

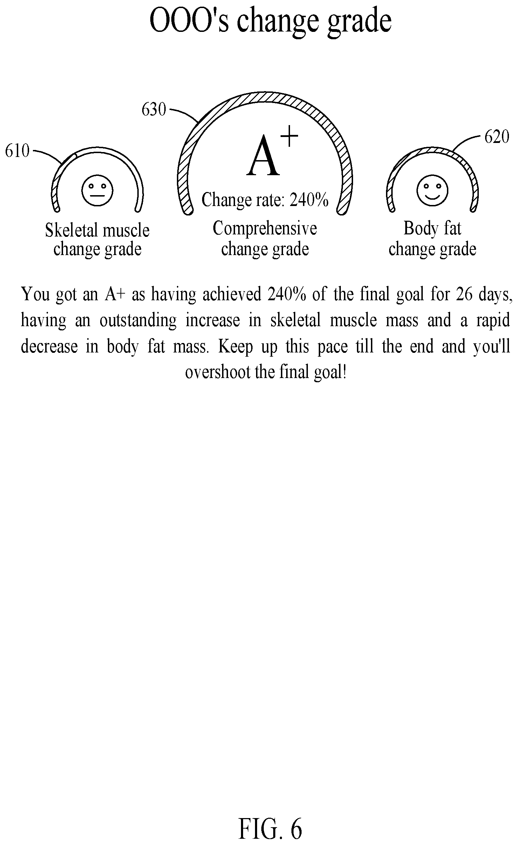

FIG. 6 illustrates a user interface displaying index values according to an example embodiment. Referring to FIG. 6, a user interface displaying index values for comparing body composition change performances between users having different body compositions is illustrated.

A method of displaying an index value may include transmitting an instruction signal to an output device such that the output device may display index values for comparing body composition change performances between users having different body compositions. The index value may be calculated based on a first period corresponding to a body composition change of the user and a second period required for a pre-selected user group to achieve the body composition change.

The method of displaying an index value may be performed by transmitting any one of a plurality of instruction signals to the output device. The plurality of instruction signals may include a first instruction signal to display a pre-calculated index value, a second instruction signal including at least one of the first period and the second period and to calculate the index value, and a third instruction signal including the first period and to calculate the second period corresponding to the first period.

Referring to the example embodiment of FIG. 6, the method of displaying an index value may include transmitting an instruction signal such that the output device may display a first graphical object 610 corresponding to a skeletal muscle change grade. For example, the skeletal muscle change grade may indicate an index value for a change pace of skeletal muscle mass according to Equation 1 or 3 described above. The first graphical object 610 may be modified in various colors, positions, and sizes according to the skeletal muscle mass change pace of the user to intuitively indicate the state of the user.

Further, the method of displaying an index value may include transmitting an instruction signal such that the output device may display a second graphical object 620 corresponding to a body fat change grade. Likewise, the body fat change grade may indicate an index value for a change pace of body fat mass according to Equation 1 or 3 described above.

Further, the method of displaying an index value may include transmitting an instruction signal such that the output device may display a third graphical object 630 for a comprehensive change grade based on the index value for the skeletal muscle mass change pace and the index value for the body fat mass change pace. The example embodiment of FIG. 6 shows a case where the result of evaluating the body composition of the user is a grade of A+, which, however, should not be construed as limiting or restricting other example embodiments.

As another example embodiment, the index value shown through the user interface may be calculated based on a first body composition change achieved by the user for a first period and a second body composition change predicted to be achievable by the pre-selected user group for the first period. In this case, the method of displaying an index value may be performed by transmitting any one of a plurality of instruction signals to the output device. The plurality of instruction signals may include any one of a first instruction signal to display a pre-calculated index value, a second instruction signal including at least one of the first body composition change and the second body composition change and to calculate the index value, and a third instruction signal including the first body composition change and to calculate the second body composition change based on the first body composition change. The index value of the present example embodiment may be calculated as a value obtained by dividing the first body composition change by the second body composition change.

The method of displaying an index value may further include obtaining body composition data of the user according to a pre-defined data transaction scheme, updating the first body composition change of the user achieved for the first period based on the received body composition data, and calculating, as the index value, a value obtained by dividing the second body composition change by the first body composition change.

FIG. 6 merely shows an exemplary user interface, and various changes may be made thereto in the process of implementing the idea of the present example embodiment, and any modified example embodiments may also be included in the spirit of the present invention. As an alternative example, an index value generating apparatus may output an index value for a body composition change rate of the user, and display the index value for the body composition change rate together with the plurality of graphical objects 610, 620, and 630. The present example embodiment displays the body composition change pace using a grade (for example, A+). However, displaying the body composition change pace using a number such as a score or a ranking may also be included in the scope of the present example embodiment.

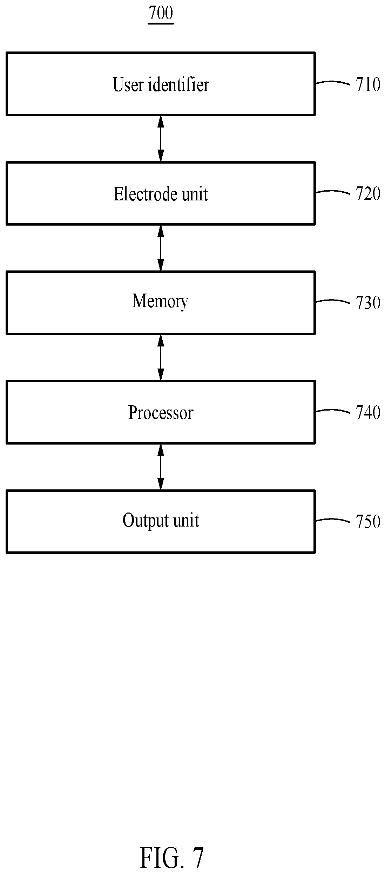

FIG. 7 illustrates a body composition measurement apparatus for generating an index value according to an example embodiment. Referring to FIG. 7, a body composition measurement apparatus 700 may include a user identifier 710, an electrode unit 720, a memory 730, a processor 740, and an output unit 750. The user identifier 710 may identify a user whose body composition is measured through the body composition measurement apparatus 700 based on user information inputted through the user interface. For example, the user information may be implemented as a name, a phone number, a social security number, or a membership number of the user.

The electrode unit 720 may be contacted with the body of the identified user to measure the current body composition data of the identified user. Hereinafter, the current body composition data refers to body composition data measured within a predefined or predetermined time from the point in time at which the user contacts the electrode unit 720. Further, previous body composition data refers to body composition data measured a predetermined time (for example, 1 hour or a day or a week) before the point in time at which the current body composition data is measured.

The memory 730 may store body composition data of a plurality of users measured for a predetermined period. For example, the memory 730 may store first previous body composition data, second previous body composition data, and current body composition data of a user A and first previous body composition data and current body composition data of a user B.

The processor 740 may update a body composition change of the identified user achieved for a first period based on previous body composition data and current body composition data of the identified user received from the memory 730. Further, the processor 740 may calculate a second period required for a pre-selected user group to achieve the body composition change.

The output unit 750 may generate an index value for comparing body composition change performances between users having different body compositions based on the first period and the second period. As a non-limiting example, the output unit 750 may be implemented as an output device such as a display or a printer.

In another example embodiment, the processor 740 of the body composition measurement apparatus 700 may update a first body composition change of the identified user achieved for the first period, based on the previous body composition data and the current body composition data of the identified user received from the memory 730. Further, the processor 740 may calculate a second body composition change predicted to be achievable by the pre-selected user group for the first period according to a big data analysis result. In this case, the output unit 750 may generate the index value for comparing body composition change performances of users having different body compositions based on the first body composition change and the second body composition change.

As described above, it is obvious to those skilled in the art that the method of generating or displaying an index value based on Equation 1, Equation 2, Equation 3, and Equation 4 may also apply to the body composition measurement apparatus 700 and is included in the scope of the present example embodiment.

The units described herein may be implemented using a hardware component, a software component and/or a combination thereof. A processing device may be implemented using one or more general-purpose or special-purpose computers, such as, for example, a processor, a controller and an arithmetic logic unit (ALU), a DSP, a microcomputer, an FPGA, a programmable logic unit (PLU), a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciate that a processing device may include multiple processing elements and multiple types of processing elements. For example, a processing device may include multiple processors or a processor and a controller. In addition, different processing configurations are possible, such as parallel processors.

The software may include a computer program, a piece of code, an instruction, or some combination thereof, to independently or uniformly instruct or configure the processing device to operate as desired. Software and data may be embodied permanently or temporarily in any type of machine, component, physical or virtual equipment, computer storage medium or device, or in a propagated signal wave capable of providing instructions or data to or being interpreted by the processing device. The software also may be distributed over network-coupled computer systems so that the software is stored and executed in a distributed fashion. The software and data may be stored by one or more non-transitory computer-readable recording mediums.

The methods according to the above-described example embodiments may be recorded in non-transitory computer-readable media including program instructions to implement various operations of the above-described example embodiments. The media may also include, alone or in combination with the program instructions, data files, data structures, and the like. The program instructions recorded on the media may be those specially designed and constructed for the purposes of example embodiments, or they may be of the kind well-known and available to those having skill in the computer software arts. Examples of non-transitory computer-readable media include magnetic media such as hard disks, floppy disks, and magnetic tape; optical media such as CD-ROM discs, DVDs, and/or Blue-ray discs; magneto-optical media such as optical discs; and hardware devices that are specially configured to store and perform program instructions, such as read-only memory (ROM), random access memory (RAM), flash memory (e.g., USB flash drives, memory cards, memory sticks, etc.), and the like. Examples of program instructions include both machine code, such as produced by a compiler, and files containing higher-level code that may be executed by the computer using an interpreter. The above-described devices may be configured to act as one or more software modules in order to perform the operations of the above-described example embodiments, or vice versa.

A number of example embodiments have been described above. Nevertheless, it should be understood that various modifications may be made to these example embodiments. For example, suitable results may be achieved if the described techniques are performed in a different order and/or if components in a described system, architecture, device, or circuit are combined in a different manner and/or replaced or supplemented by other components or their equivalents.

**Claims**

1. A computing device for generating an index value for comparing body composition change performances between users having different body compositions, the computing device comprising:

   a communication unit configured to obtain body composition data of a user from a body composition measurement apparatus that is in connection thereto or to update a body composition change of the user achieved for a first period;
   a processor configured to calculate a second period required for a pre-selected user group to achieve the body composition change; and
   an output unit configured to generate an index value for comparing body composition change performances between users having different body compositions based on the first period and the second period.

2. The computing device of claim 1, wherein the processor is configured to select a first user group having a body fat decrease or a muscle mass increase in a pre-selected storage device, and to calculate a second user group in the first user group as the pre-selected user group, the second user group whose age, gender, and height have a predetermined or higher correlation with those of the user.

3. The computing device of claim 2, wherein the processor is configured to calculate, as the second period, a period required to reach a body fat mass and a muscle mass after the first period from a body fat mass and a muscle mass of the user before the first period, based on body composition change data of the second user group.

4. The computing device of claim 1, wherein when the output unit is implemented as a display, the display is configured to display the generated index value on a screen.

5. The computing device of claim 1, wherein when the output unit is implemented as a printer, the printer is configured to provide a result printout including the generated index value.

6. A method of displaying an index value for comparing body composition change performances between users having different body compositions, the method comprising:

transmitting an instruction signal to an output device such that the output device displays an index value for comparing body composition change performances between users having different body compositions, wherein the index value is calculated based on a first period corresponding to a body composition change of a user and a second period required for a pre-selected user group to achieve the body composition change.

7. The method of claim 6, wherein the instruction signal comprises any one of:

a first instruction signal to display a pre-calculated index value;
a second instruction signal including at least one of the first period and the second period and to calculate the index value; and
a third instruction signal including the first period and to calculate the second period corresponding to the first period.

8. The method of claim 7, wherein
the second period is calculated as a period required for the pre-selected user group to reach a body fat mass and a muscle mass after the first period from a body fat mass and a muscle mass of the user before the first period,
the pre-selected user group is pre-selected as a second user group whose age, gender, and height have a predetermined or higher correlation with those of the user, in a first user group having a body fat decrease or a muscle mass increase, and
a value obtained by dividing the second period by the first period is calculated as the index value.

9. The method of claim 6, comprising:

obtaining body composition data of the user according to a pre-defined data transaction scheme;
updating a body composition change of the user achieved for the first period based on the obtained body composition data; and
calculating, as the index value, a value obtained by dividing the second period by the first period.

10. The method of claim 9, further comprising:

calculating, as the second period, a period required for the pre-selected user group to reach a body fat mass and a muscle mass after the first period from a body fat mass and a muscle mass of the user before the first period.

11. The method of claim 9, wherein the obtaining of the body composition data of the user comprises any one of:

receiving the body composition data of the user from a body composition measurement apparatus that is in connection;
receiving the body composition data of the user being inputted through a user interface; and
extracting the body composition data of the user from a designated code image.

12. A body composition measurement apparatus for generating an index value for comparing body composition change performances of users having different body compositions, the body composition measurement apparatus comprises:

a user identifier configured to identify a user whose body composition is to be measured through the body composition measurement apparatus based on user information being inputted through a user interface;
an electrode unit configured to measure current body composition data of the identified user;
a memory configured to store body composition data of a plurality of users measured for a predetermined period;
a processor configured to update a body composition change of the identified user achieved for a first period based on previous body composition data and the current body composition data of the identified user received from the memory and to calculate a second period required for a pre-selected user group to achieve the body composition change; and

an output unit configured to generate an index value for comparing body composition change performances between users having different body compositions based on the first period and the second period.

13. A computing device for generating an index value for comparing body composition change performances between users having different body compositions, the computing device comprising:

a communication unit configured to obtain body composition data of a user from a body composition measurement apparatus that is in connection thereto or to update a first body composition change of the user achieved for a first period;

a processor configured to calculate a second body composition change predicted to be achievable by a pre-selected user group for the first period according to a big data analysis result; and

an output unit configured to generate an index value for comparing body composition change performances between users having different body compositions based on the first body composition change and the second body composition change.

14. The computing device of claim 13, wherein the processor is configured to select a first user group having a body fat decrease or a muscle mass increase in a pre-selected storage device, and to calculate a second user group in the first user group as the pre-selected user group, the second user group whose age, gender, and height have a predetermined or higher correlation with those of the user.

15. The computing device of claim 14, wherein the output unit is configured to generate, as the index value, a value obtained by dividing the first body composition change by the second body composition change.

16. A method of displaying an index value for comparing body composition change performances between users having different body compositions, the method comprising:

transmitting an instruction signal to an output device such that the output device displays an index value for comparing body composition change performances between users having different body compositions,

wherein the index value is calculated based on a first body composition change of a user achieved for a first period and a second body composition change predicted to be achievable by a pre-selected user group for the first period.

17. The method of claim 16, wherein the instruction signal comprises any one of:

a first instruction signal to display a pre-calculated index value;

a second instruction signal including at least one of the first body composition change and the second body composition change and to calculate the index value; and

a third instruction signal including the first body composition change and to calculate the second body composition change based on the first body composition change.

18. The method of claim 17, wherein the index value is calculated as a value obtained by dividing the first body composition change by the second body composition change.

19. The method of claim 16, comprising:

obtaining body composition data of the user according to a pre-defined data transaction scheme;

updating the first body composition change of the user achieved for the first period based on the received body composition data; and

calculating, as the index value, a value obtained by dividing the second body composition change by the first body composition change.

20. The method of claim 19, wherein the obtaining of the body composition data of the user comprises any one of:

receiving the body composition data of the user from a body composition measurement apparatus that is in session connection;

receiving the body composition data of the user being inputted through a user interface; and

extracting the body composition data of the user from a designated code image.

21. A body composition measurement apparatus for generating an index value for comparing body composition change performances of users having different body compositions, the body composition measurement apparatus comprises:

a user identifier configured to identify a user whose body composition is to be measured through the body composition measurement apparatus based on user information being inputted through a user interface;

an electrode unit configured to measure current body composition data of the identified user;

a memory configured to store body composition measurement values of a plurality of users measured for a predetermined period;

a processor configured to update a first body composition change of the identified user achieved for a first period based on previous body composition data and the current body composition data of the identified user received from the memory and to calculate a second body composition change predicted to be achievable by a pre-selected user group for the first period according to a big data analysis result; and

an output unit configured to generate an index value for comparing body composition change performances between users having different body compositions based on the first body composition change and the second body composition change.

<u>100</u>

| Communication unit | ～110 |

↕

| Processor | ～120 |

↕

| Output unit | ～130 |

FIG. 1

| | Before weight control (First point in time) | | Target value | | Before weight control (Second point in time) | | Target value | | First period | Second period | | First index value | Third period | Second index value |
|---|---|---|---|---|---|---|---|---|---|---|---|---|---|---|
| | Body fat | Skeletal muscle | Body fat | Skeletal muscle | Body fat | Skeletal muscle | Body fat | Skeletal muscle | | Body fat | Skeletal muscle | | | |
| User A | 30kg | 35kg | -6kg | 0kg | 28kg | 35kg | -2kg | 0kg | 30days | 10days | 0days | 0.33 | 60days | 17% |
| User B | 12kg | 35kg | -2kg | 0kg | 10kg | 35kg | -2kg | 0kg | 30days | 30days | 0days | 1.0 | 60days | 50% |
| User C | 8kg | 33kg | -0.5kg | 0kg | 7kg | 35kg | -1kg | 0kg | 30days | 45days | 0days | 1.5 | 60days | 67% |

FIG. 2A

| | Before weight control (First point in time) | | Target value | | Before weight control (Second point in time) | | Target value | | First period | Second period | | First index value | Third period | Second index value |
|---|---|---|---|---|---|---|---|---|---|---|---|---|---|---|
| | Body fat | Skeletal muscle | Body fat | Skeletal muscle | Body fat | Skeletal muscle | Body fat | Skeletal muscle | | Body fat | Skeletal muscle | | | |
| User A | 30kg | 35kg | -6kg | 0kg | 28kg | 35kg | -2kg | 0kg | 45days | 10days | 0days | 0.22 | 60days | 17% |
| User B | 12kg | 35kg | -2kg | 0kg | 10kg | 35kg | -2kg | 0kg | 45days | 30days | 0days | 0.67 | 60days | 50% |
| User C | 8kg | 33kg | -0.5kg | 0kg | 7kg | 35kg | -1kg | 0kg | 45days | 45days | 0days | 1.0 | 60days | 67% |

FIG. 2B

| | Change | | Actual weight control period | Midterm target change | | First index value | Whole weight control period | Final target change | | Second index value |
|---|---|---|---|---|---|---|---|---|---|---|
| | Body fat | Skeletal muscle | | Body fat | Skeletal muscle | | | Body fat | Skeletal muscle | |
| User A | -2kg | 0kg | 30days | -6kg | 0kg | 0.33 | 60days | -10kg | 0kg | 20% |
| User B | -2kg | 0kg | 30days | -2kg | 0kg | 1.0 | 60days | -6kg | 0kg | 33% |
| User C | -1kg | 0kg | 30days | -0.5kg | 0kg | 2.0 | 60days | -1kg | 0kg | 100% |

FIG. 3

| | Before weight control (First point in time) | | Before weight control (Second point in time) | | Actual change | | First period | Second period | | First index value | Body composition change grade |
|---|---|---|---|---|---|---|---|---|---|---|---|
| | Body fat | Skeletal muscle | Body fat | Skeletal muscle | Body fat | Skeletal muscle | | Body fat | Skeletal muscle | | |
| User A | 30kg | 35kg | 28kg | 38kg | -2kg | 3kg | 30days | 10days | 45days | 1.83 | A- |
| User B | 12kg | 35kg | 10kg | 36kg | -2kg | 3kg | 30days | 30days | 15days | 1.5 | B+ |
| User C | 8kg | 33kg | 7kg | 36kg | -1kg | 3kg | 30days | 45days | 90days | 4.5 | A+ |

FIG. 4

511

Receive body composition data of
user from body composition
measurement apparatus

512

Obtain body composition data of
user inputted through user interface

513

Extract body composition data of
user from designated code image

500

Index value generating apparatus

FIG. 5

# OOO's change grade

630

A$^+$

Change rate: 240%

610

620

Skeletal muscle
change grade

Comprehensive
change grade

Body fat
change grade

You got an A+ as having achieved 240% of the final goal for 26 days, having an outstanding increase in skeletal muscle mass and a rapid decrease in body fat mass. Keep up this pace till the end and you'll overshoot the final goal!

FIG. 6

<u>700</u>

```
┌─────────────────────────────────┐
│         User identifier         │  ~ 710
└─────────────────────────────────┘
                ↕
┌─────────────────────────────────┐
│         Electrode unit          │  ~ 720
└─────────────────────────────────┘
                ↕
┌─────────────────────────────────┐
│             Memory              │  ~ 730
└─────────────────────────────────┘
                ↕
┌─────────────────────────────────┐
│            Processor            │  ~ 740
└─────────────────────────────────┘
                ↕
┌─────────────────────────────────┐
│           Output unit           │  ~ 750
└─────────────────────────────────┘
```

FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1470593